# EUROPÄISCHE PATENTSCHRIFT

(11) **EP 4 309 571 B1**
(45) Veröffentlichungstag und Bekanntmachung des Hinweises auf die Patenterteilung: **20.11.2024**
(21) Anmeldenummer: 23214347.9
(22) Anmeldetag: 09.06.2021
(51) Int. Cl.: A61B 18/12, A61B 18/14, A61B 18/16, A61B 5/053, A61B 5/24, A61B 5/00

(54) **ÜBERWACHUNGSEINHEIT UND HOCHFREQUENZ-CHIRURGIE-SYSTEM MIT EINER SOLCHEN ÜBERWACHUNGSEINHEIT**
MONITORING UNIT AND HIGH-FREQUENCY SURGICAL SYSTEM COMPRISING SUCH A MONITORING UNIT
UNITÉ DE SURVEILLANCE ET SYSTÈME DE CHIRURGIE HAUTE FRÉQUENCE COMPRENANT UNE TELLE UNITÉ DE SURVEILLANCE

(30) Priorität: 12.06.2020 DE 102020003524
(43) Veröffentlichungstag der Anmeldung: 24.01.2024
(62) Teilanmeldung aus: 21734288.0
(73) Patentinhaber: FORBENCAP GmbH, 87527 Sonthofen (DE)
(72) Erfinder: Götz, Stefan, 85659 Forstern (DE); Scherz, Pascal, 80538 München (DE); Conle, Robert Ludwig, 87527 Sonthofen (DE)
(74) Vertreter: advotec.

(56) Entgegenhaltungen:
- DE-A1- 102018 114 482
- DE-B4- 112013 001 594
- US-A1- 2004 015 058
- US-A1- 2008 281 309
- US-B2- 8 864 756

## Beschreibung

Die vorliegende Erfindung betrifft eine Überwachungseinheit, die dazu eingerichtet ist, einen Patienten während eines Betriebes eines Hochfrequenz-Chirurgiegerätes zu überwachen und ein Hochfrequenz-Chirurgie-System mit einer solchen Überwachungseinheit.

Hochfrequenz-Chirurgie (kurz: HF-Chirurgie) ist mitweilen eine dominierende Form der Elektrochirurgie. Sie umfasst einen (assistierenden) Einsatz von elektrischer Energie zur (gezielten) thermisch induzierten Veränderung und/oder Zerstörung von Gewebezellen, um dadurch eine sog. Hämostase (Blutstillung), eine Gewebedurchtrennung und/oder - versiegelung (Koagulation) zu erreichen. Dabei wird hochfrequenter Wechselstrom (bspw. 0,3 bis 4 MHz) über Elektroden (bzw. Applikatoren) in das zu behandelnde Gewebe geleitet. In dem Gewebe kommt es durch einen in diesem vorhandenen elektrischen Gewebewiderstand zu einer thermischen Gewebewechselwirkung, auf die im Weiteren nochmals eingegangen wird.

Ein wesentlicher Vorteil der hochfrequenzchirurgischen Gewebetrennung gegenüber herkömmlicher, chirurgischer Schneidetechnik, bspw. mittels Skalpells oder Schere, ergibt sich dadurch, dass zeitgleich mit dem Schnitt eine Blutungsstillung durch Verschluss der betroffenen Gefäße erreicht werden kann. Ferner begründen sich in einer Verhütung einer Keimverschleppung, einer mechanischen Gewebeschonung sowie in einer Möglichkeit eines endoskopischen Einsatzes der HF-Chirurgie-Technik weitere, nicht abschließend aufgezählte Vorteile dieser Technologie.

Aufgrund der Vorteile für Patient und Operateur sowie der stetig steigenden Nachfrage aus unterschiedlichsten medizinischen Bereichen ist der Bedarf an HF-chirurgischen Technologien sowie deren stetige Weiterentwicklung im medizinisch-technischen Fokus und begründet eine unstreitig vielseitige gewerbliche Anwendbarkeit dieser Technologie.

Aus elektrotechnischer Hinsicht kann der sog. Stromdichte (physikalische Einheit: Ampere pro cm²) bei der HF-Chirurgie-Technologie eine Schlüsselrolle zugewiesen werden, wobei die elektrische Stromdichte umgangssprachlich kennzeichnet, wie dicht zusammengedrängt ein elektrischer Strom fließt. Damit kennzeichnet die Stromdichte bspw. auch die Belastung eines elektrischen Leiters (im Falle der HF-Chirurgie repräsentiert durch tierisches oder menschliches Gewebe) durch einen elektrischen (Wechsel-) Strom. Die Stromdichte ist definiert als das Verhältnis der Stromstärke I zu einer dem Strom zur Verfügung stehenden Querschnittsfläche A.

In Bezug auf die HF-Chirurgie kann ein (gewünschter) Schneide und/oder Koagulationseffekt (Koagulation = Ausfällung, Ausflockung oder Gerinnung eines Stoffes) des behandelten Gewebes nur erreicht werden, wenn die Stromdichte in dem mittels der Hochfrequenztechnik behandelten Gewebeabschnitt ausreichend groß (üblicherweise zwischen 1 bis 6 Ampere pro cm²) ist. Die Stromdichte nimmt quadratisch mit einer Entfernung r von dem Applikationszentrum ab (J ∼ 1/r²). Hingegen nimmt eine (messbare) Temperaturerhöhung bei Voraussetzung homogener Gewebeeigenschaften mit der 4. Potenz zu der Entfernung r vom Applikationszentrum ab (ΔT ∼1/r⁴).

Beim Gewebeschneiden mittels hochfrequenten Wechselstromes lassen sich vorzugsweise über kleinflächige, messer- oder nadelförmige Elektroden lokal begrenzt hohe Stromdichten in dem zu schneidenden Gewebe(-abschnitt) erzeugen. Das durch den hochfrequenten Wechselstrom therapierte Gewebe erhitzt sich dabei blitzartig über 100 °C, so dass ein entstehender Dampfdruck die Zellmembranen der Gewebezellen explosionsartig zerreißt. Der dabei hervorgerufene elektrisch isolierende Dampf zwischen Elektrode und Gewebe verhindert in der Folge den ungehinderten Ohm'schen Stromfluss in das Gewebe, so dass sich eine Spannung (sog. Schneidespannung) zwischen Elektrode und Gewebe aufbaut, durch welche eine Funkenbildung zwischen Elektrode und Gewebe hervorgerufen wird. Ein weiterer Energieeintrag erfolgt sodann über Funken. In den nur wenige µm großen Fußpunkten (r_{F} = 10 bis 20 µm) der Funken treten dabei extrem hohe Energiedichten auf, unter deren Einfluss die einbezogenen Gewebezellen vaporisieren. Dabei spielt es keine Rolle, ob der Vorgang offen unter Umgebungsbedingungen, unter einer Schutzatmosphäre oder in einer elektrisch nichtleitenden Flüssigkeit erfolgt.

Welche Wirkung ein HF-Strom in dem behandelten Gewebe hervorruft, ist im Wesentlichen durch dessen Einwirkdauer, Spannungshöhe und einen Grad der Amplitudenmodulation vorgegeben. Die Frequenz (0,3 bis 5 MHz) spielt hingegen keine tragende Rolle. Eine wichtige Kenngröße stellt der sog. Crestfaktor (engl.: crest, Scheitelpunkt) dar. Er beschreibt das Verhältnis von Spitzenwert (Scheitelwert) zu Effektivwert einer elektrischen Wechselgröße und gibt einen Hinweis darauf, wie stark ein Strom in seiner Amplitude moduliert ist. So hat z. B. eine sinusförmige Wechselspannung mit einem Effektivwert (U_{eff}) von 230 V_{eff} (Netzspannung) einen Spitzenwert (Uₚ) von ca. 325 Vₚ, so dass der Crestfaktor (C_{F}) hier als 1,41 (^2) ist. Bei konstanter Ausgangsleistung muss demnach bei einem Strom mit hohem Crestfaktor auch die Ausgangsspannung größer sein.

Das "Herzstück" der HF-Chirurgie-Technik wird durch einen sogenannten Hochfrequenzgenerator gebildet, wobei sich die hintergründige Technik insbesondere getrieben durch mannigfaltige mikroprozessorische und regelungstechnische Weiterentwicklungen innerhalb der vergangenen Jahrzehnte stetig verbessert hat. In modernen Hochfrequenzgeneratoren wird ein generatorspeisender Netzstrom in einen (für die HF-Chirurgie notwendigen) Hochfrequenzstrom umzuwandeln. Dabei wird die hochfrequente Ausgangsleistung der HF-Chirurgiegeräte getrieben durch sicherheitstechnische Standardisierung und technische Weiterentwicklung (z. B. durch die Normengebung (IEC 60601-2-2)) stetig verringert und ist auch eine Obergrenze von 400 W begrenzt. Ferner empfiehlt die IEC 60601-2-2 Arbeitsfrequenzen oberhalb von 300 kHz und unterhalb von 5 MHz anzuwenden.

Im Zuge der sicherheitsmotivierten Standardisierung weisen moderne Hochfrequenzgenerator weiterhin eine Mehrzahl an internen Überwachungseinrichtungen, wie z. B. ein sog. Neutralelektroden-Monitoring, Überdosierungsschutzschaltungen, optische und akustische Aktivierungsanzeigen und eine sog. HF-Ableitstromkompensation auf, wodurch während der Anwendung ein höheres Maß an Sicherheit für den Patienten und Operateur gewährleistet werden kann.

Trotz der beschriebenen Vorteile sowie stetigen (sicherheits-) technischen Weiterentwicklungen besteht bei HF-Chirurgie-Applikationen weiterhin die Problematik von (zum Teil schwersten) Verbrennungen und/oder Verätzungen des Patienten an Gewebebereichen, die oftmals von dem eigentlichen Applikationszentrum der HF-Chirurgiegerät (d.h. dem Operationsumfeld) entfernt liegen, z. B. an Füßen, Händen, Teilen des oberen und unteren Rückens, etc. Diese Verbrennungen und/oder Verätzungen (bei zusätzlicher Interaktion mit Desinfektionsmittel) sind auf Nebeneffekte eines Energieeintrages an hochfrequenter elektrischer Energie in das Gewebe des Patienten zurückzuführen, auf die nachfolgend nochmals eingegangen wird.

Als potenzielle "Quelle" der unerwünschten Verbrennungen und/oder Verätzungen kommt oftmals eine unsachgemäße Handhabung der im Vorfeld vor der Operation stattfindenden Vorbereitung des Patienten, bspw. durch unsachgemäße Lagerung (bzw. Aufbringung) des Patienten auf einem Operationstisch oder -stuhl, in Betracht. Dem wird zwar versucht, durch die Definition und Normstellung einer Vielzahl von sicherheitsrelevanten Anwendungsregeln, dieser Fehlbedienung bzw. fehlerhaften Handhabung (menschliches Fehlverhalten) entgegenzuwirken, was je nach Operationsumgebung zwar Erfolg zeigt, dennoch das Auftreten von Verbrennungen und Verätzungen des Patienten nicht vollständig unterbindet.

Die unerwünschte Verletzung des Patienten an Gewebeabschnitten, an denen kein operativer Eingriff erfolgt, löst dabei oftmals nachträgliche Schadensersatzforderungen (z. B. in Form von Schmerzensgeld und dem Ersatz von Behandlungskosten) in zum Teil nicht unerheblichem Umfang aus, die von Seiten des Klinikbetreibers durch Inanspruchnahme eines entsprechenden Versicherungsschutzes (und damit einhergehenden Mehrausgaben) oder aus Eigenkapitalaufbringung abzufedern bzw. auszugleichen sind.

Die physikalische Ursache der Verbrennungen bzw. Verätzungen an Gewebeteilen, die oftmals von dem eigentlichen Operationsgeschehen entfernt sind, ist bspw. auf eine elektrisch nicht vollständig isolierte Lagerung des Patienten und eine damit einhergehende unerwünschte und unkontrollierte Ableitung von Strömen (entlang des Weges des geringsten Widerstandes) zurückzuführen. Ferner kann bei einem (zufälligen) Kontakt eines Gewebeteils des Patienten (z. B. eines Fußes) mit Gerätschaften (Schläuchen, etc.) in der Peripherie des Patienten eine nicht vorhergesehene Leitungsbrücke entstehen, über die der durch das HF-Chirurgie-Gerät eingebrachte Strom (zusätzlich) ausgeleitet werden kann. Diese Leitungsbrücken können dabei auch an im Niederfrequenzbereich elektrisch isolierenden Materialien entstehen und sind zumeist auf kapazitive Kopplungseffekte zurückzuführenden. Somit erwartet der Anwender (Operateur) bspw. von einem Plastikschlauch dessen elektrisch isolierende Wirkung, obgleich derselbe Plastikschlauch bei einem Inkontakttreten mit hochfrequenter elektrischer Energie elektrisch leitend wirkt.

Eine Ausprägung des Auftretens der unerwünschten Verbrennungen und/oder Verätzungen des Patienten unterscheidet sich zudem aufgrund der eigesetzten HF-Technologie, wobei zwischen monopolarer und bipolarer Anwendung unterschieden wird. Für weitere Informationen wird auf Kramme, Rüdiger, ed. Medizintechnik: Verfahren-Systeme-Informationsverarbeitung. Springer-Verlag, 2016, Kapitel 32 verwiesen.

Die monopolare HF-Chirurgie-Technik weist eine aktive Elektrode und eine neutrale Flächenelektrode auf, die jeweils an den HF-Generator angeschlossen sind. An der Aktivelektrode werden die für die HF-Chirurgie notwendigen physikalischen Effekte (Schneiden, Koagulieren) erzeugt. Die im Vergleich zur Aktivelektrode großflächigere Neutralelektrode wird bspw. an einem Oberschenkel oder einem Oberarm des Patienten platziert. Eine Kontaktfläche zwischen der Neutralelektrode und der Haut(-oberfläche) des Patienten ist dabei möglichst groß (= möglichst geringe Stromdichte). Der durch die Aktivelektrode eingeleitete, hochfrequente Wechselstrom wird im Idealfall vollständig über die Neutralelektrode abgeleitet. Der restliche Körper des Patienten ist hingegen im Idealfall elektrisch isoliert gelagert.

Bei der bipolaren HF-Chirurgie-Technik ist hingegen sowohl die aktive als auch die neutrale Elektrode in einem Instrument, z. B. einer bipolaren Pinzette mit gegeneinander isolierten Schenkeln (Branchen), integriert. Der hochfrequente Wechselstrom fließt bei dieser Technik über die aktive Elektrode in das Gewebe hinein und über die neutrale Elektrode zum HF-Generator zurück. Das bedeutet, dass der Wechselstrom in einem eng umschriebenen Gewebebereich zwischen den beiden Elektrodenspitzen fließt, weshalb die bipolare Technik insbesondere bei neurologischen Feinpräparationen ein höheres Maß an Sicherheit im Vergleich zu der monopolaren Technik gewährleistet. Zudem ist im Vergleich zu der monopolaren Technik die Gefahr von unerwünschten Verbrennungen und/oder Verätzungen an dem Patienten, z. B. durch Berührung leitfähiger Gegenstände während der Operation, eher gering, jedoch nicht ausgeschlossen.
Weiterer Stand der Technik ist der US 2008 / 0 281 309 A1, der DE 10 2018 114 482 A1, der US 8 864 756 B2, der US 2009 / 0 234 352 A1 und der US 7 837 680 B2 zu entnehmen.

In Anbetracht des Vorgenannten ist es eine Aufgabe der vorliegenden Erfindung eine Überwachungstechnologie bereitzustellen, durch die eine Anzahl unerwünschter Verbrennungen und/oder Verätzungen an Gewebebereichen, die nicht von dem eigentlichen operativen Eingriff betroffen sind, verringert werden kann.

Eine hier nicht beanspruchte Lösung dieser Aufgabe kann durch eine Überwachungseinheit erfolgen, dazu eingerichtet ist, einen Patienten während eines Betriebes eines Hochfrequenz-Chirurgiegerätes zu überwachen. Das Hochfrequenz-Chirurgiegerät ist dazu ausgebildet, biologisches Gewebe (des Patienten) mittels elektrischer Hochfrequenz-Energie zu trennen und/oder zu koagulieren. Die Überwachungseinheit weist Messelektroden, die in einer Peripherie des Patienten angeordnet sind, und eine Auswerte- und Steuereinheit auf, die dazu eingerichtet ist, eine vorbestimmte Mess-Wechselspannung oder einen vorbestimmten Mess-Wechselstrom in die Messelektroden einzuprägen, und eine zwischen den Messelektroden abfallende Impedanz zu messen, und einen zeitlichen Verlauf der Impedanz und/oder eine zeitliche Änderung derselben zu überwachen. Vorzugsweise ist die Auswerte- und Steuereinheit dazu eingerichtet, ein Warnsignal zu erzeugen, wenn eine Relativänderung der Impedanz in dem Verlauf der Impedanz einen vorbestimmten ersten Grenzwert unterschreitet oder überschreitet und/oder die Impedanz einen vorbestimmten zweiten Grenzwert unterschreitet.

Die Überwachungseinheit hat den Vorteil, dass mittels der Impedanz-Messung und Überwachung der Peripherie des Patienten dieser vor unerwünschten (bspw. aufgrund von unkontrollierten Ableitströmen und/oder Kriechströmen hervorgerufenen) Verbrennungen und/oder Verätzungen an eigentlich nicht von dem operativen Eingriff betroffenen Gewebeteilen (z. B. an einem Fuß, einer Hand und/oder an Teilen des Rückens des Patienten) geschützt werden kann.

Die Impedanz-Messung ermöglicht sozusagen eine Vorhersage einer sich anbahnenden Verbrennung und/oder Verätzung, auf die mittels Ausgabe des Warnsignals hingewiesen werden kann, so dass bspw. der Operateur oder Hilfspersonal manuell eingreifen kann. Alternativ oder ergänzend kann ein geregeltes Abschalten des Hochfrequenz-Chirurgiegerätes mittels der Auswerte- und Steuereinheit oder mittels eines Hochfrequenz-Generators erfolgen.

Das Hochfrequenz-Chirurgiegerät weist vorzugsweise eine Aktivelektrode und eine Neutralelektrode auf, wobei zumindest die Aktivelektrode mit einem vorzugsweise vorhandenen Hochfrequenz-Generator verbunden ist. Die Aktivelektrode ist dazu ausgebildet, biologisches Gewebe mittels der elektrischen Hochfrequenz-Energie zu trennen und/oder zu koagulieren, und die Neutralelektrode ist dazu eingerichtet, die elektrische Hochfrequenz-Energie aus dem biologischen Gewebe abzuleiten. Vorzugsweise ist die Neutralelektrode ebenfalls mit dem Hochfrequenz-Generator verbunden.

Ein unkontrollierter hochfrequenter Stromfluss über einen Gewebeabschnitt bzw. ein Gewebeteil in der Peripherie der Operationszone kann bspw. auf eine unsachgemäße Lagerung des Patienten auf dem Operationstisch hervorgerufen werden. Hierdurch kann es bspw. durch eine (zufällige) Berührung eines im Hochfrequenzbereich leitfähigen Operationsequipments (Beatmungs- oder Absaug-Schlauch, Teil des Operationstisches) zur Ausbildung einer sog. Leitungsbrücke kommen, über die zumindest teilweise oder (im schlimmsten Fall) gesamthaft der über die Aktivelektrode eingebrachte Wechselstrom abfließen kann. Im Idealfall sollte der über die Aktivelektrode eingebrachte Schneidstrom ausschließlich über die Neutralelektrode abfließen. Mittels der Impedanz-Messung können diese Leitungsbrücken quasi in Echtzeit ermittelt werden.

Unter der Formulierung "in einer Peripherie des Patienten" wird verstanden, dass die Messelektroden (vorzugsweise eine Vielzahl von Messelektroden, jedoch zumindest mehr als eine Messelektrode) in einer unmittelbaren und/oder mittelbaren Umgebung des Operationszentrums und/oder an dem oder um den Patienten angeordnet sind. D.h. mit anderen Worten, dass die Messelektroden bspw. auf einer Operationsauflage, auf der der Patient während der Operation gelagert wird, bspw. über eine (vorzugsweise gesamte) Auflagefläche (regelmäßig) verteilt angeordnet sein können.

Alternativ oder ergänzend können die Messelektroden an Operationsequipment (Beatmungs- und Absaug-Schläuche, Verkabelungen etc.) in der Peripherie des Patienten angeordnet sein. Es ist bevorzugt, dass die Messelektroden auf Oberflächen angeordnet sind, die in direktem Kontakt mit einem Körperteil bzw. der Haut des Patienten sind (bspw. der Rückenbereich durch direkte Auflage auf dem Operationstisch) und/oder die potenziell mit einem Gewebeabschnitt (bzw. einem Körperteil) des Patienten in Kontakt treten können (bspw. bei unsachgemäßer Lagerung ein Abgleiten der Hand des Patienten von dem Operationstisch und ein darauffolgendes Inkontakttreten der Hand mit bspw. einem Beatmungsschlauch).

Zudem kann es zu Situationen kommen, bei denen elektrisch leitfähige (Körper-) Flüssigkeit(en) (Blut, Exkremente, etc.) unkontrolliert austreten und beim Inkontakttreten mit Materialoberflächen, wie z. B. der Operationsliege oder dem Operationstisch, die Leitfähigkeit zwischen diesen Materialien und der darauf liegenden Haut schlagartig erhöhen und somit an diesen Stellen Leitungsbrücken entstehen.

Kommt es zu einem unerwarteten bzw. unkontrollierten Stromfluss über eine Leitungsbrücke, nimmt eine an den Messelektroden im Passivzustand (d.h. bei vollständiger Stromableitung über die Neutralelektrode ohne einen Stromfluss über eine weitere Leitungsbrücke) gemessene Impedanz schlagartig ab (Impedanz |Z| = U_{eff} / l_{eff}). Überschreitet oder unterschreitet die Relativänderung der Impedanz in dem gemessenen Verlauf der Impedanz den vorbestimmten ersten Grenzwert (welcher bspw. mittels einer Vielzahl von Testmessungen ermittelt wurde) und/oder unterschreitet die Impedanz einen vorbestimmten zweiten Grenzwert (bspw. eine derart niedrige Impedanz, dass mit einer sofortigen Verbrennung des betreffenden Gewebes zu rechnen ist), wird sodann vorzugsweise das Warnsignal erzeugt und/oder das Hochfrequenz-Chirurgiegerät mittels der Auswerte- und Steuereinheit dazu veranlasst, abzuschalten.

Unter der Formulierung "Relativänderung der Impedanz" wird jeweiliger Gradient (Anstieg oder Abfall) der Impedanz über die Zeit verstanden. Die Auswerte- und Steuereinheit ist dazu eingerichtet, den Gradienten aus dem zeitlichen Verlauf der Impedanz zu bestimmen (Vf = ∂Z / ∂t, mit Z = Impedanz [Ω] und t = Zeit [s]).

Vorzugsweise sind die Messelektroden derart ausgebildet, dass sie gemeinsam als die Neutralelektroden agieren.

Unter der "Mess-Wechselspannung" oder dem "Mess-Wechselstrom" wird vorliegend der zur Impedanz-Messung in die Messelektroden eingebrachte Strom bzw. die Spannung verstanden. So kann zwischen den Messelektroden eine vorzugsweise durchgängige Impedanz-Messung vorgenommen werden, d. h., bspw. wird ein Mess-Wechselstrom einprägen und eine Spannung gemessen oder eine Mess-Wechselspannung wird eingeprägt und ein entsprechender Strom wird gemessen. Wenn die Impedanz zwischen jeweils zwei Messelektroden entweder stark abfällt (∂Z/∂t) (erster Grenzwert) oder unter eine Schwelle (zweiter Grenzwert) fällt, deutet dies bspw. auf ein Austreten von leitenden Flüssigkeiten oder auf ein Berühren einer Hautstelle mit einem elektrisch leitenden Gegenstand hin und erlaubt eine Reaktion.

Es sei noch erwähnt, dass die Vielfalt der unterschiedlichen Gewebearten (u. a. Muskel, Fett, etc.) und deren Zustände (blutend, trocken, inniger oder loser Elektrodenkontakt, etc.), an denen ein Hochfrequenz-(Schneid-)Strom appliziert wird oder über die ein hochfrequenter Strom abfließt, ein breites Impedanzspektrum abbilden (ca. 50-1000 Ω).

Die Impedanz-Messung mittels der Messelektroden grenzt sich insbesondere gegenüber bestehenden Sicherheitseinrichtungen, wie z. B. Neutralelektroden-Monitoring, Überdosierungsschutzschaltungen, optische und akustische Aktivierungsanzeigen und eine HF-Ableitstromkompensation, welche allesamt in einem Hochfrequenzgenerator integriert sein können, grundlegend ab. Die Abgrenzung besteht insbesondere darin, dass die Überwachungseinrichtung von den Sicherheitskonzepten des Hochfrequenzgenerators vorzugsweise als eigenständige bzw. separate Überwachungs- und/oder Sicherheitseinheit entkoppelt ist. Somit können vorzugsweise auf der gesamten Auflagefläche des Patientenkörpers potenzielle Gefahrenstelle für sich anbahnende Verbrennungen ermittelt und, bspw. durch ein Warnsignal, frühzeitig behoben werden. Somit können auch Störungen, die auf menschliches Fehlverhalten zurückzuführen sind, überwacht werden, was mit der in den Hochfrequenzgenerator integrierten Sicherheitssysteme nicht oder lediglich bedingt möglich ist.

Eine weitere hier nicht beanspruchte Lösung dieser Aufgabe kann durch eine Überwachungseinheit erfolgen, die dazu eingerichtet ist, einen Patienten während eines Betriebes eines Hochfrequenz-Chirurgiegerätes zu überwachen, wobei das Hochfrequenz-Chirurgiegerät dazu ausgebildet ist, biologisches Gewebe (des Patienten) mittels Hochfrequenz-Energie zu trennen und/oder zu koagulieren. Die Überwachungseinheit weist Messsensoren und eine Auswerte- und Steuereinheit auf, wobei die Messsensoren dazu eingerichtet sind, einen Parameter zu erfassen, der während des Trennens und/oder Koagulierens des biologischen Gewebes aufgrund der Hochfrequenz-Energie hervorgerufen wird. Vorzugsweise ist die Auswerte- und Steuereinheit dazu eingerichtet, auf Basis des Parameters ein Warnsignal zu erzeugen.

Bei dieser Lösung erfolgt die Messung bzw. das Erkennen eines unbeabsichtigten und/oder unkontrollierten Stromabflusses über einen Körperteil des Patienten, bei dem zum Teil schwere Verbrennungen und/oder Verätzungen entstehen können, mittels Messsensoren, d.h. nicht direkt durch das Messen von Impedanz-Werten. Vielmehr ist es möglich, sich anbahnende Verbrennungen und/oder Verätzungen auch mittels weiterer technischer Verfahren möglichst für die gesamte Köperoberfläche des Patienten zu überwachen und sich anbahnende Verbrennungen frühzeitig zu ermitteln. Je nach physikalischem Messprinzip (z. B. über einen Temperatur-Messsensoren, etc.) erfasst die Auswerte und Steuereinheit einen Parameter anhand dessen indirekt auf eine unerwünschten Stromabfluss über einen Körperteil des Patienten geschlossen werden kann.

Weiterhin wird die Aufgabe durch ein Hochfrequenz-Chirurgie-System gelöst, das einen Hochfrequenz-Generator aufweist, der dazu eingerichtet ist, eine Hochfrequenz-Energie zu erzeugen. Ferner weist das System ein Hochfrequenz-Chirurgiegerät mit einer Aktivelektrode und einer Neutralelektrode auf, wobei zumindest die Aktivelektrode mit dem Hochfrequenz-Generator verbunden ist. Die Aktivelektrode ist dazu ausgebildet, biologisches Gewebe mittels der elektrischen Hochfrequenz-Energie zu trennen und/oder zu koagulieren, und die Neutralelektrode ist dazu eingerichtet, die elektrische Hochfrequenz-Energie aus dem biologischen Gewebe abzuleiten. Ferner weist das Hochfrequenz-Chirurgie-System eine Ausgestaltung der erfindungsgemäßen Überwachungseinheit auf.

Weiterhin ist es vorteilhaft, die Überwachungseinheit bspw. mit einer oder mehreren optischen Sensoren (z. B. Kameras) zu koppeln, um so eine Live-Abbildung des Operationsgeschehens zu erhalten. Die durch die Kamera(s) erfassten (3D-) Bilder können vorzugsweise mit den gemessenen Größen (Impedanz oder Parameter) derart überlagert werden, dass eine visuelle Lokalisierung des unerwünschten Stromflusses bzw. die Stelle, an der es zu einer (zusätzlichen) Strombrücke kommt, bspw. mittels eines Displays ermöglicht ist.

Ein Vorteil des ersten und zweiten Aspektes ist, dass ein einfacher Retrofit ermöglicht wird, so dass sämtliche bestehende Systeme sicherheitstechnisch nachrüstbar sind. Zudem ist es von Vorteil, dass durch das Überwachungssystem ein deutlich höheres Maß an Sicherheit für den Operateur, jedoch insbesondere für den Patienten erreicht werden kann. Fehlverhalten oder eine Missachtung von Vorschriften bei der Vorbereitung der Operation, z. B. bei der Lagerung des Patienten können somit durch die Überwachsungseinheit zumindest teilweise kompensiert werden Somit kommt es zu weniger Verbrennungen und zu einer Entlastung der Versicherer und mithin zu einer unmittelbaren und mittelbaren Kosteneinsparung.

Bei dem Hochfrequenz-Chirurgiegerät handelt es sich vorzugsweise um ein Hochfrequenz-Elektroskalpell. Das Hochfrequenz-Chirurgiegerät kann in monopolarer oder bipolarer Bauweise ausgeführt sein. Ferner kann es sich bei dem Hochfrequenz-Chirurgiegerät allgemein gesprochen um ein Gerät handeln, bei welchem hochfrequente elektrische Energie zu Operationszwecken appliziert wird.

In einer Ausgestaltung sind die Messelektroden an mehreren Komponenten in der Peripherie des Patienten angeordnet, so dass die Auswerte- und Steuereinheit dazu eingerichtet ist, eine räumliche Impedanz-Verteilung in der Peripherie des Patienten zu ermitteln.

Ein Vorteil dieser Ausgestaltung ist, dass somit vorzugsweise sämtliches Equipment, dass sich in der Nähe des Patienten bzw. dessen Körper befinden, auf eine potenzielle Ausbildung einer Leitungsbrücke bzw. Strombrücke überwacht werden kann. Somit ist es möglich, vorzugsweise sämtliche für Verbrennungen anfällige Körperstellen des Patienten indirekt durch Impedanz-Messungen an vorzugsweise sämtlichen Komponenten im Operationsbereich zu überwachen.

In einer weiteren Ausgestaltung weist die Überwachungseinheit ferner eine Anzeige auf und die Auswerte- und Steuereinheit ist dazu eingerichtet, das bevorzugte Warnsignal in Form einer räumlichen Position und Lage eines Ortes in der Peripherie des Patienten, an dem der erste Grenzwert überschritten oder unterschritten und/oder der zweite Grenzwert unterschritten wird, auf der Anzeige anzuzeigen.

Ein Vorteil dieser Ausgestaltung besteht darin, dass der Operateur oder ein Hilfspersonal den Ort des unkontrollierten Stromabflusses, der nicht über die dafür vorgesehene Neutralelektrode erfolgt, möglichst exakt zu lokalisieren. Somit ist ein gezieltes Eingreifen möglich und eine sich anbahnende Verbrennung kann verhindert bzw. zumindest frühzeitig gestoppt werden, so dass kollaterale Schädigungen des betroffenen Hautgewebes verhindert werden.

In einer weiteren Ausgestaltung ist die Auswerte- und Steuereinheit ferner dazu eingerichtet, das Hochfrequenz-Chirurgiegerät bei Überschreiten oder Unterschreiten des ersten Grenzwertes und/oder bei Unterschreiten des zweiten Grenzwertes abzuschalten.

Ein Vorteil dieser Ausgestaltung besteht darin, dass der Operateur bzw. Hilfspersonal nicht manuell tätig werden muss, falls eine weitere Leitungsbrücke detektiert wird, sondern das Hochfrequenz-Chirurgiegerät automatisch abgeschaltet wird, um so Verbrennungen zu verhindern. Bei Abschaltung wird der Stromfluss durch die Aktivelektrode und somit das Einbringen von hochfrequenter elektrischer Energie abrupt gestoppt, so dass Verbrennungen ausbleiben.

Es sei erwähnt, dass der Mechanismus des Abschaltens vorzugsweise manuell ausgeschaltet werden kann, da es insbesondere bei Notoperationen immer wieder zu Situationen kommen kann, bei denen elektrisch leitfähige (Körper-) Flüssigkeit(en) (Blut, Exkremente, etc.) unkontrolliert austreten und beim Inkontakttreten mit Materialoberflächen, wie z. B. der Operationsliege oder dem Operationstisch, die Leitfähigkeit zwischen diesen Materialien und der darauf liegenden Haut schlagartig erhöhen und somit Leitungsbrücken entstehen. Obgleich dieser Faktor unerwünschte Verbrennungen hervorruft, ist es in diesen Ausnahmesituationen aufgrund der akuten Gefahr für das menschliche Leben entscheidender, dass ein Betrieb des Hochfrequenz-Operationsgerätes sichergestellt ist.

In einer weiteren Ausgestaltung bilden die Messelektroden ein Muster aus räumlich verteilten jeweils benachbarten und vorzugsweise voneinander elektrisch isolierten Flächenelektroden, und die Auswerte- und Steuereinheit ist dazu eingerichtet, zwischen jeweils zwei benachbarten Flächenelektroden die Impedanz zu messen.

Ein Vorteil dieser Ausgestaltung besteht darin, dass eine (möglichst genaue) Lokalisierung des Bereiches, an der eine Leitungsbrücke entstanden ist, möglich ist und bspw. auf einem Bildschirm markiert werden kann (beispielsweise ein 3D-Bild der Operationsliege oder des Operationstisches, auf dem die entsprechenden Bereiche jeweils mit einer roten Punktewolke markiert werden). Alternativ oder ergänzend kann bspw. gleichzeitig ein akustischer Alarm und/oder eine Unterbrechung des HF-Operationsgerätes erfolgen.

In einer weiteren Ausgestaltung sind die Messelektroden in Form einer Interdigitalstruktur ausgestaltet.

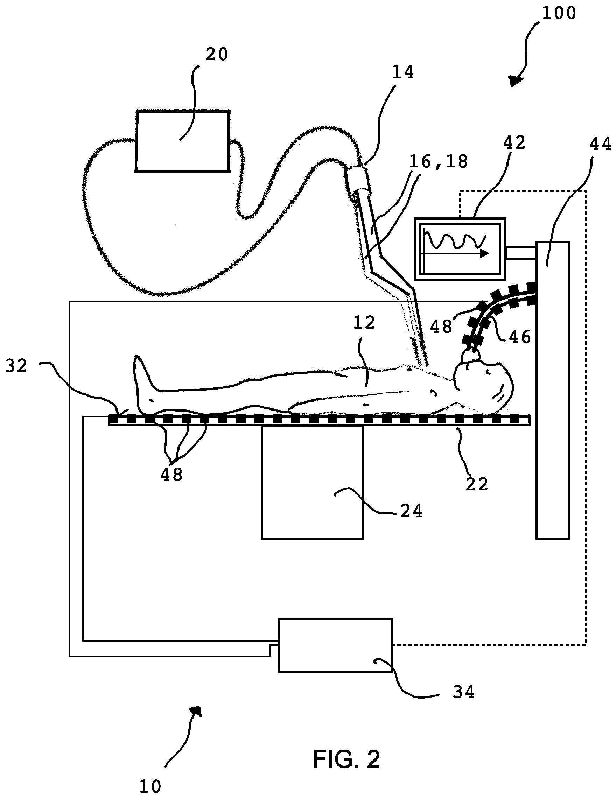

Beispielhafte Interdigitalstruktur können folgenden der wissenschaftlichen Literatur, beispielsweise Fig. 2 aus Tsai, Chiu, Chou (2015). Optimal Design of SAW Gas Sensing Device by Using Improved Adaptive Neuro-Fuzzy Inference System. IEEE Access, Ausgabe 3. S. 420-429 entnommen werden.

Interdigitalstruktur weisen fingerartige Strukturen auf. Diese Strukturen werden auch als Interdigitalelektroden bezeichnet und greifen zumeist kammartig ineinander, ohne sich zu berühren. Die fingerartigen Strukturen bestehen bspw. aus einem Metall.

In einer weiteren Ausgestaltung weist die vorbestimmte Mess-Wechselspannung oder der vorbestimmte Mess-Wechselstrom eine Frequenz von 1 kHz bis 10 kHz auf und/oder entspricht nicht einer Frequenz, mit der das Hochfrequenz-Chirurgiegerät betrieben wird.

Die Messfrequenz ist also vorzugsweise AC, und besonders bevorzugt nicht Frequenzbestandteil des Schneidstromes, der in die Aktivelektrode gespeist wird. Dies ist vorteilhaft für das Signal-Rausch-Verhältnis und ermöglicht eine vereinfachte, störungsfreiere Analyse bzw. Auswertung der Mess-Impedanz. Die bevorzugte Frequenz von 1 kHz bis 10 kHz ermöglicht eine vereinfachte Erkennung von "Leckagen".

Es sei jedoch erwähnt, dass grundsätzlich auch Frequenzen gewählt werden können, die zumindest in der Nähe der Frequenz des Schneidstromes sind. In diesem Fall sollte jedoch eine entsprechende Rausch-Filterung oder synchrone Demodulation (beispielsweise als Heterodyn- und vorzugsweise als Homodyn-Detektion), mittels derer Messsignal-Störungen herausgefiltert werden, erfolgen.

In einer weiteren Ausgestaltung sind die Messelektroden derart angeordnet, dass sie sich in elektrisch leitendem Kontakt mit einer oder mehreren Körperstellen und/oder einer Kleidung des Patienten befinden.

Ein Vorteil dieser Ausgestaltung ist, dass durch den elektrisch leitenden Kontakt, die zwischen den Messelektroden gemessene Impedanz bereits den elektrischen Widerstand der einen oder mehreren Körperstellen umfasst, und somit bei sich anbahnenden Verbrennungen, z. B. bei einem plötzlichen Austreten von Flüssigkeiten während der Operation, eine sich verändernde Leitfähigkeit der Haut indirekt über die herabfallende Impedanz bestimmen lässt. Vorzugsweise sind die Messelektroden in direktem Kontakt mit der Hautoberfläche, die sich auf dem Operationstisch befindet. Alternativ können die Messelektroden auch in Kontakt mit einer Bekleidung sein, die der Patient während der Operation trägt. Ein solches Bekleidungsstück, bspw. ein Operationskittel, kann bei einem unerwarteten Austritt von (Körpereigener-) Flüssigkeit oder Desinfektionsmittel Feuchtigkeit aufnehmen, bis hin zu durchnässen, wodurch ein plötzlicher Impedanzabfall hervorgerufen wird.

In einer weiteren Ausgestaltung sind die Messelektroden adhäsiv mit der einen oder mehreren Körperstellen des Patienten elektrisch leitend verbunden.

Ein Vorteil dieser Ausgestaltung, dass somit ein unmittelbarer Kontakt zwischen der Hautoberfläche und den Messelektroden sichergestellt ist. Somit wird die Qualität der Impedanz-Messung erhöht, da Störsignale (z. B. durch Luftbrücken) vermieden werden.

In einer weiteren Ausgestaltung ist eine Anzahl an Messelektroden zumindest größer als eine Anzahl der zu überwachenden einen oder mehreren Körperstellen des Patienten.

Ein Vorteil dieser Ausgestaltung besteht in der Möglichkeit einer exakteren bzw. genauere Lokalisierung, einer Korrektur von Fehlsignalen sowie einer Redundanz, für einen Fall, in dem einige der Messelektroden einen schlechten elektrischen Kontakt zum Patienten aufweisen.

In einer weiteren Ausgestaltung sind die Messelektroden auf einer Oberfläche einer Patientenauflage angeordnet oder in der Oberfläche der Patientenauflage integriert.

Die Messelektroden können bspw. unmittelbar auf der Oberfläche des Operationstisches angeordnet oder sogar in der Oberfläche integriert sein. Alternativ können die Messelektroden auch in einem netzartigen Überzug integriert sein, der vor der Operation über den Operationstisch gezogen wird. Ferner ist es möglich, dass die Messelektroden bspw. in einem schlauchartigen Netzüberzug angeordnet sind, der jeweils über die Beatmungs- und oder Absaugschläuche übergezogen werden kann. Die Patientenauflage kann bspw. ein Operationsstuhl oder ein Operationstisch sein.

Ein Vorteil dieser Ausgestaltung besteht darin, dass ein unmittelbarer, d.h. direkter, Kontakt zwischen den Messelektroden und der Hautoberfläche des Patienten sichergestellt werden kann.

In einer weiteren Ausgestaltung sind die Messelektroden in einen abnehmbaren Überzug einer Patientenauflage oder einer Matte eingebracht.

Ein Vorteil dieser Ausgestaltung ist der einfache Retrofit für bestehende Systeme, da der Operationstisch lediglich mit dem Überzug der Patientenauflage, in dem die Messelektroden bzw. Messsensoren angeordnet sind, überzogen werden kann. Zudem ist eine leichte Reinigung des Überzuges möglich. Der Überzug kann bspw. aus einem Stoff und/oder einem Plastik hergestellt sein.

Unter dem Begriff "eingebracht" wird verstanden, dass die Messelektroden bzw. Messsensoren vorzugsweise in dem Überzug eingewebt, eingenäht, eingeklebt, aufgeklebt oder in sonstiger Weise befestigt sind. Vorzugsweise ist bei der Ausgestaltung des Überzugs auf den Komfort des Patienten zu achten, um so Quetschungen oder Druckstellen, insbesondere während mehrstündiger Operationen, zu vermeiden.

In einer weiteren Ausgestaltung weisen die Messelektroden jeweils elektrisch leitende Fäden auf, die vorzugsweise in dem Überzug eingewebt sind.

Ein Vorteil dieser Ausgestaltung ist die Einfachheit ihrer Ausführung. So kann bspw. ein Stoff, in den metallische Fäden voneinander beabstandet eingewebt sind, als Überzug verwendet werden, wobei jeder der metallischen Fäden dann als eine Messelektrode fungiert und die Impedanz-Messung jeweils zwischen zwei Fäden, die nicht notwendigerweise benachbart sein müssen, erfolgt.

In einer weiteren Ausgestaltung sind die Messelektroden messtechnisch differentiell in Serie zueinander oder parallel zueinander geschaltet oder miteinander induktiv oder kapazitiv gekoppelt, und fungieren vorzugsweise in ihrer Gesamtheit als eine Neutralelektrode des Hochfrequenz-Chirurgiegerätes, die die elektrische Hochfrequenz-Energie aus dem biologischen Gewebe ableitet.

Ein Vorteil dieser Ausgestaltung besteht darin, dass die Messelektroden je nach verwendeter Elektrodenart in beliebiger Weise miteinander elektrisch verschaltet werden können. Die konkrete Art der Verschaltung ist dann stets in Bezug auf die Auswertung mittels der Auswerte- und Steuereinheit maßgeblich, da sich je nach Art der Verschaltung die Berechnung der Messgrößen verändert. Vorzugsweise dienen die Messelektroden in ihrer Gesamtheit als die Neutralelektrode, was den Vorteil hat, dass auf den Einsatz einer zusätzlichen Neutralelektrode verzichtet werden kann. Über die Neutralelektrode erfolgt dann ein geregeltes, kontrolliertes Abfließen des mittels der Aktivelektrode in das Gewebe eingebrachten Hochfrequenzstromes.

In einer weiteren Ausgestaltung erfolgt eine Einprägung der vorbestimmten Mess-Wechselspannung oder des vorbestimmten Mess-Wechselstromes in die Messelektroden auf Basis einer Vierleitermessung.

Die Vierleitermessung wird bei der Messung von elektrischen Widerständen mit einem Vierleiteranschluss eingesetzt, wenn Leitungs- und Anschlusswiderstände die Messung verfälschen können. Bei der Vierleiter-Messanordnung fließt über zwei der Leitungen ein bekannter elektrischer Messstrom durch den Widerstand (das zwischen den Messelektroden liegende Gewebe). Die am Widerstand abfallende Spannung wird hochohmig über zwei weitere Leitungen abgegriffen und mit einem Spannungsmessgerät gemessen; der zu messende Widerstand wird nach dem ohmschen Gesetz berechnet.

In einer weiteren Ausgestaltung sind die Messelektroden jeweils als Elektrodenpaare elektrisch miteinander verschaltet, so dass die Einprägung der vorbestimmten Mess-Wechselspannung oder des vorbestimmten Mess-Wechselstromes und die Messung der Impedanz jeweils paarweise erfolgt, wobei jede einzelne Messelektrode der Messelektroden jeweils mit einer beliebigen anderen Messelektrode der Messelektroden dynamisch paarbar ist, und wobei die Messung der Impedanz pro Messelektrodenpaar vorzugsweise zeitlich nacheinander oder gleichzeitig erfolgt.

Ein Vorteil dieser Ausgestaltung besteht darin, dass eine segmentweise bzw. abschnittsweise Messung der Impedanz möglich ist. Ein weiterer Vorteil ist die Möglichkeit einer kostengünstigen Lokalisierung möglicher Verbrennungen. Der oder die wahrscheinlichsten Orte möglicher Verbrennungen können visuell, bspw. auf einem Bildschirm, dargestellt und/oder markiert werden.

Um Messkanäle und/oder Messelektroden "einzusparen", können Messkanäle an mehreren voneinander entfernten Orten verwendet und mit anderen Nachbarkanälen verschaltet werden. Da bei einer Leckage sehr wahrscheinlich großflächiger auch Nachbarelektrodenpaare betroffen werden, kann man auf diese Weise über das "Anschlagen" (d.h., das Messen einer stark abfallenden Impedanz) zusammen mit Nachbarelektroden weiterhin den Ort der Verbrennung relativ genau bestimmen. Zugleich wird eine höhere Auflösung erreicht als wenn man nur weniger Kanäle einsetzte.

In einer weiteren Ausgestaltung ist die Auswerte- und Steuereinheit dazu eingerichtet, einen Realteil und/oder Imaginärteil der über die Messelektroden abfallenden Impedanz zu messen und/oder eine Amplitude und Phase der Mess-Wechselspannung oder des Mess-Wechselstromes zu messen.

Die Messung der Amplitude und Phase der Mess-Wechselspannung oder des Mess-Wechselstromes entspricht einer Bestimmung des Imaginär- und des Realteils der Impedanz.

In einer weiteren Ausgestaltung sind die Messsensoren Temperatursensoren, vorzugsweise thermoaktive Elemente, die in der Peripherie des Patienten angeordnet und dazu eingerichtet sind, eine Temperatur als den Parameter zu erfassen. Ferner ist in dieser Ausgestaltung die Auswerte- und Steuereinheit dazu eingerichtet, das bevorzugte Warnsignal zu erzeugen, wenn eine Relativveränderung der Temperatur einen vorbestimmten ersten Schwellwert überschreitet.

Ein Vorteil dieser Ausgestaltung besteht darin, dass ein Entstehen einer unerwünschten Leitungsbrücke aufgrund eines Inkontakttretens eines Körperteils mit einem elektrisch leitfähigen Material nicht unmittelbar über eine Impedanz-Messung, die ggf. auch Rauscheffekten unterliegen kann, erfolgt, sondern mittelbar aufgrund der zur den Stromfluss über die Leitungsbrücke hervorgerufenen Temperaturerhöhung. Der erste Schwellwert ist bspw. eine Grenztemperatur ab der es potenziell zur Entstehung von Verbrennungen kommen kann.

Es sei erwähnt, dass diese Messtechnik eine gewisse Trägheit des Entstehens einer Verbrennung voraussetzt, d. h., dass es zumindest einige Sekunden zur Ausbildung einer Verbrennung dauert.

Die zu verwendenden Temperatursensoren weisen hingegen möglichst kaum thermische Trägheit auf, weshalb sich insbesondere thermoelektrische Sensortypen (z. B. Thermo-Elemente, Positive Temperature Coefficient (PTC)-Thermistor, Negative Temperature Coefficient Thermistor (NTC)) als Sensortyp eignen, da diese neben einer geringen thermischen Trägheit ebenfalls günstig in ihrer Herstellung sind und für Differenztemperaturmessungen eine ausreichende Genauigkeit aufweisen.

In einer weiteren Ausgestaltung sind die Temperatursensoren an mehreren Komponenten in der Peripherie des Patienten angeordnet, so dass die Auswerte- und Steuereinheit dazu eingerichtet ist, eine räumliche Temperaturverteilung in der Peripherie des Patienten zu ermitteln.

Ein Vorteil dieser Ausgestaltung besteht darin, dass vorzugsweise eine flächige, vorzugsweise räumliche, Temperaturverteilung der Operationsumgebung zumindest abschnittsweise erfasst werden kann. Besonders bevorzugt weist die Überwachungseinheit ergänzend eine oder mehrere thermisch aktive Messkameras, z. B. in Form von Infrarot-Kameras auf, mittels derer die Temperaturverteilung des sichtbaren Operationsbereiches erfasst werden kann. Durch Überlagerung der Messergebnisse der Temperatursensoren sowie der thermischen Kameras kann so vorzugsweise eine dreidimensionale Temperaturverteilung der Operationsumgebung erstellt werden. Das eigentliche Operationszentrum, in dem das Schneiden mittels des Hochfrequenz-Chirurgiegerätes erfolgt, wird vorzugsweise aus der Temperaturverteilung ausgeblendet, da in diesem Beriech ohnehin verbrennungskritische Temperaturen hervorgerufen werden.

Erfindungsgemäß sind die Messsensoren jeweils magnetische und/oder elektrische Antennen, die dazu eingerichtet sind, als den Parameter jeweils elektromagnetische Messsignale aus der Peripherie des Patienten zu erfassen, die durch die Hochfrequenz-Energie während des Betriebes des Hochfrequenz-Chirurgiegerätes erzeugt werden. Ferner ist erfindungsgemäß die Auswerte- und Steuereinheit dazu eingerichtet, mittels Lösens einer mathematischen Inversproblematik aus den elektromagnetischen Messsignalen eine Stromdichteverteilung im Raum (d.h. von dem Patienten und dessen (vorzugsweise unmittelbarer) Umgebung) zu berechnen. Die erfassten, elektromagnetischen Messsignale verhalten sich bei magnetischen Antennen proportional zu 1/r • ∂l/∂t, bei elektrischen Antennen proportional zu 1/Abstand • ∂V/∂t; mit r = Abstand, l = Strom, t = Zeit, V = Spannung.

Aus den mittels der Antennen aufgefangenen Messsignalen kann über die Lösung eines Inversproblems die Strom(-dichte)-verteilung im Raum berechnet werden. Es wird also danach gefragt, wie die Stromdichteverteilung im Operationsbereich, welche durch das Hochfrequenz-Operationsgerät aufgrund des Schneidstromes hervorgerufen wird, aussehen müsste, um die mittels der durch die Antennen erfassten Messsignale zu erzeugen.

Vorzugsweise werden unter dem Begriff "Antennen" bspw. magnetische Antennen (z. B. Ferritkern-Antennen), elektrische Antennen (z. B. Dipol-Antennen, bspw. λ/4, λ /2, Sub-λ/2 Antennen und/oder Kurzantennen mit erweiterten Kapazitäten (d. h. einer virtuellen Verlängerung) und/oder Magnetfeld-Sensoren (z. B. Hall-Sensoren, SQUID-Sensoren, Magnetoresistiv-Sensoren und/oder Fluxgate-Magnetometer) verstanden.

Somit ist es möglich quasi ein 3D-Tomographiebild der Stromdichteverteilung im Raum zu erhalten. Bei Überschreiten einer (vorbestimmten) Schwelle oder beim Auftreten (Berechnen) von Strömen in Bereichen, in denen keine Ströme fließen sollten, kann ein Warnsignal erzeugt werden. Das Warnsignal kann bspw. in akustischer, optischer und/oder taktiler Form ausgegeben werden. Alternativ oder ergänzend ist es möglich, das Hochfrequenz-Operationsgerät zu unterbrechen bzw. abzuschalten.

Ferner ist es von Vorteil, wenn die errechnete dreidimensionale Stromdichteverteilung bildhaft dargestellt und vorzugsweise mit Bildern einer 3D-Stereo-Kamera (z. B. vom Hersteller Kinect, Microsoft, Intel, NDI) derart überlagert, dass diese Stromdichte-Informationen bspw. an einer teiltransmissiv 3D-Abbildung des Patienten visualisiert sind. Diese Visualisierung ist aufgrund heutiger Grafikkarten (GPUs) und programmierbares Logikgattern bzw. integrierter Schaltkreise (FPGAs) in Echtzeit möglich.

Ein weiterer Vorteil dieser Ausgestaltung besteht darin, dass ein Retrofit auf relativ einfache und kostengünstige Weise realisiert werden kann. Bspw. können in einem Operationssaal mehrere elektrische oder magnetische Antennen in den jeweiligen Ecken des Raumes montiert werden. Die jeweils eingefangenen Messsignale werden gesamthaft an die Auswerte- und Steuereinheit geführt. Die Auswerte und Steuereinheit kann bspw. in dem Hochfrequenzgenerator integriert oder als separate Recheneinheit von diesem getrennt angeordnet sein.

Unter dem Begriff "Inversproblematik" wird ein mathematisches Problem umfasst, bei dem man von einer beobachteten oder gewünschten Wirkung (hier die gemessenen Signale der Antennen) eines Systems auf die der Wirkung zugrundeliegende Ursache (hier die Stromdichteverteilung) zurückschließt. Es wird für weitere Informationen auf Frank Natterer: The Mathematics of Computerized Tomography. Society for Industrial and Applied Mathematics, Philadelphia 2001. ISBN 0-89871-493-1 verwiesen.

Es sei erwähnt, dass die Ermittlung der Stromdichteverteilung mittels Lösung der Inversproblematik sich grundsätzlich auch dazu eignet, bspw. während einer TUR-Anwendung frühzeitig zu erkennen. Die transurethrale Resektion der hypertrophen Prostata (TUR-P) sowie die Resektionen in der Blase (TUR-B) in der Urologie oder die transzervikale Resektion des Endometriums in der Gynäkologie sind Standardverfahren. Dabei wird über ein (starres) Resektoskop transurethral Blasen- oder Prostatagewebe HF-chirurgisch monopolar unter elektrisch isolierender Zuckerlösung oder bipolar unter elektrisch leitfähiger NaCl-Lösung mit einer Schlingenelektrode reseziert. Erfolgt dabei eine nicht ausreichende Spülung, können H₂O-Moleküle zu H₂ und O₂ dissoziieren und sich am Harnblasendach ansammeln. Wird in diesem Gasgemisch reseziert, kann es zu einer Explosion kommen. Mittels der dreidimensionalen Stromdichteverteilung kann eine sich anbahnende Dissoziation möglichst frühzeitig erkannt werden.

Ferner ist die Ermittlung der Stromdichteverteilung auch für weitere hochfrequenzchirurgische Operationsverfahren, bei denen die Operation unter Verwendung eines Endoskops erfolgt, vorteilhaft.

In einer weiteren Ausgestaltung ist die Auswerte- und Steuereinheit dazu eingerichtet, das Warnsignal zu erzeugen und/oder das Hochfrequenz-Chirurgiegerät abzuschalten, wenn die Stromdichte einer oder mehreren vorbestimmten Positionen im Raum einen vorbestimmten zweiten Schwellwert überschreitet.

Ein Vorteil dieser Ausgestaltung besteht darin, dass der Operateur das HF-Chirurgiegerät nicht manuell abschalten muss, sondern ein solches Abschalten automatisiert ist.

Unter dem "zweiten Schwellwert" wird vorliegend bspw. eine kritische Stromdichte, in einem anderen Bereich als dem Operationszentrum, in dem man keine hohen Stromdichten erwarten würde, verstanden.

Die "eine oder mehreren Positionen" können bspw. ein Teil oder mehrere Teile eines Rückens des Patienten, mit welchen der Patient auf der Operationsliege aufliegt, und/oder ein oder mehrere Teil(e) eines Fußes oder einer sonstigen Gliedmaße des Patienten repräsentieren.

Besonders bevorzugt ist ferner eine Überwachungseinheit gemäß einem weiteren Aspekt der Erfindung, wobei die Überwachungseinheit die dazu eingerichtet ist, einen Patienten während eines Betriebes eines Hochfrequenz-Chirurgiegerätes zu überwachen, wobei das Hochfrequenz-Chirurgiegerät dazu ausgebildet ist, biologisches Gewebe mittels Hochfrequenz-Energie zu trennen und/oder zu koagulieren, wobei die Überwachungseinheit magnetische und/oder elektrische Antennen aufweist, die dazu eingerichtet sind, jeweils elektromagnetische Messsignale aus einer Peripherie des Patienten zu erfassen, die durch die Hochfrequenz-Energie während des Betriebes des Hochfrequenz-Chirurgiegerätes erzeugt werden. Ferner weist die Überwachungseinheit eine Auswerte- und Steuereinheit, die dazu eingerichtet ist, mittels Lösens einer mathematischen Inversproblematik aus den elektromagnetischen Messsignalen eine Stromdichteverteilung im Raum zu berechnen.

Es versteht sich, dass die vorstehend genannten und die nachstehend noch zu erläuternden Merkmale nicht nur einzeln oder in der jeweils angegebenen Kombination, sondern auch in anderen Kombinationen verwendbar sind.

Die erfindungsgemäße Überwachungseinheit sowie das erfindungsgemäße System sind grundsätzlich bei allen medizinisch-technischen Geräten, Verfahren und/oder Behandlungstechniken einsetzbar, bei denen hochfrequente elektrische Energie am Patienten appliziert wird.

Zudem versteht es sich, dass grundsätzlich auch synergetische technische Effekte durch den Einsatz der Überwachungseinheit sowie deren jeweilige Ausgestaltungen entstehen und vorteilhaft genutzt werden können. So kann es bspw. vorteilhaft sein, die Impedanz-Messung zusammen mit dem (dreidimensionalen) Stromdichte-Mapping mittels elektrischer oder magnetischer Antennen und/oder mit der Temperaturüberwachung mittels thermoelektrischer Sensorik einzusetzen, d.h. mehrere technisch unterschiedlich funktionierende Überwachungseinheiten einzusetzen. Dies kann bspw. sicherheitsseitige Vorteile (aufgrund von Ausfallsicherheiten durch Redundanzen) als auch messtechnische Vorteile bieten, da physikalisch sich voneinander unterscheidende Messergebnis zur jeweiligen Gegenprüfung miteinander vereinen lassen, was eine Steigerung der Präzision des Überwachungsergebnisses zur Folge haben kann. Weiterhin sei erwähnt, dass die erfindungsgemäße Überwachungseinheit ebenfalls im veterinärmedizinischen Bereich, d.h. bei der Operation von Tieren, zum Einsatz kommen, und dass der zuvor genannte Patient nicht notwendigerweise menschlicher, sondern auch tierischer Natur sein kann.

Ausführungsbeispiele der Erfindung sind in den Zeichnungen dargestellt und werden in der nachfolgenden Beschreibung näher erläutert. Es zeigen:
- Fig. 1: eine schematische Ansicht eines ersten Ausführungsbeispiels eines Hochfrequenz-Chirurgie-Systems
- Fig. 2: eine schematische Ansicht eines zweiten Ausführungsbeispiels eines Hochfrequenz-Chirurgie-Systems
- Fig. 3: eine schematische Ansicht eines zeitlichen Impedanz-Verlaufes;
- Fig. 4.: eine schematische Ansicht eines Ausführungsbeispiels der Messelektroden;
- Fig. 5: eine schematische Ansicht eines zweiten Ausführungsbeispiels der Messelektroden;
- Fig. 6: eine schematische Ansicht eines dritten Ausführungsbeispiels der Messelektroden; und
- Fig. 7: eine schematische Ansicht eines dritten Ausführungsbeispiels eines erfindungsgemäßen Hochfrequenz-Chirurgie-Systems.

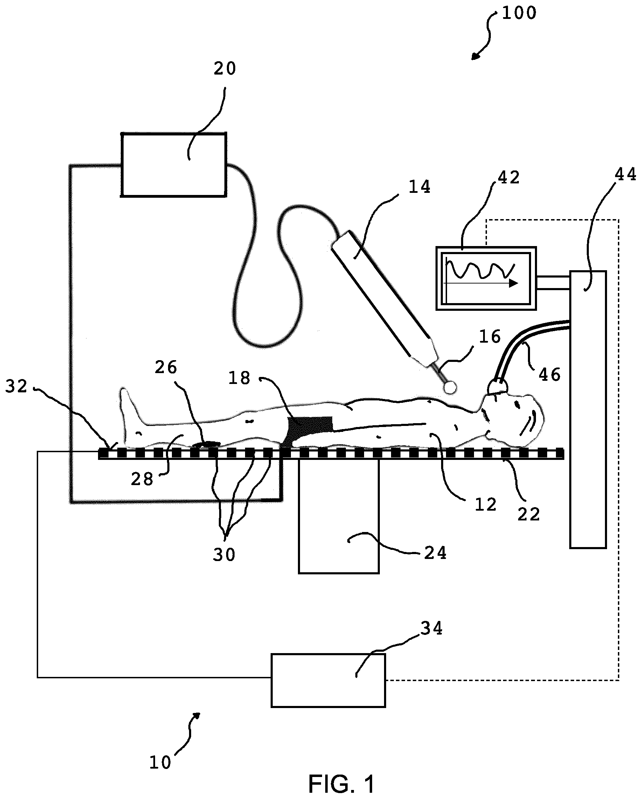

Fig. 1 zeigt ein erstes Ausführungsbeispiel eines Hochfrequenz-Chirurgie-Systems in schematischer Ansicht. Das Hochfrequenz-Chirurgie-Systems ist in seiner Gesamtheit mit der Bezugsziffer 100 gekennzeichnet. Das Hochfrequenz-Chirurgie-System weist ein Ausführungsbeispiel einer Überwachungseinheit 10 auf.

Die Überwachungseinheit 10 ist dazu eingerichtet, einen Patienten 12 während eines Betriebes eines Hochfrequenz-Chirurgiegerätes 14 zu überwachen. Das Hochfrequenz-Chirurgiegerät 14 ist vorliegend ein Elektroskalpell in monopolarer Bauweise.

Das Hochfrequenz-Chirurgiegerät 14 weist eine Aktivelektrode 16 und einen Neutralelektrode 18 auf. Die Aktivelektrode 16 ist dazu ausgebildet, biologisches Gewebe des Patienten 12 mittels einer elektrischen Hochfrequenz-Energie bzw. durch Einspeisung eines hochfrequenten Wechselstroms zu durchtrennen und/oder zu koagulieren. Die Neutralelektrode 18 ist dazu eingerichtet, die elektrische Hochfrequenz-Energie aus dem biologischen Gewebe abzuleiten. Vorliegend ist die Neutralelektrode 18 als Flächenelektrode auf einem Oberschenkel des Patienten 12 adhäsiv aufgebracht.

Die Aktivelektrode 16 und die Neutralelektrode 18 sind über ein oder mehrere Kabel elektrisch mit einem Hochfrequenzgenerator 20 gekoppelt. Der Hochfrequenzgenerator 20 ist dazu eingerichtet, einen netzseitig (z. B. über einen 230 V, 16 A; oder einen 380 V, 32 A Anschluss) bezogenen elektrischen Strom leistungselektronisch (bspw. mittels Frequenzwandlern) in einen für den Betrieb des Hochfrequenz-Chirurgiegerätes notwendigen Hochfrequenz-Wechselstrom umzuwandeln. Die Frequenz dieses Schneidstroms ist vorzugsweise oberhalb von 300 kHz und unterhalb von 5 MHz. Die Neutralelektrode 18 kann in anderen Ausführungsarten auch an anderen Körperteilen des Patienten 12 angebracht sein, oder alternativ durch einen Erdungsanschluss eines Operationstisches 22 gebildet sein.

Der Operationstisch 22 ist vorliegend als horizontale Patientenauflage ausgeführt, der über einen monolithischen Sockel 24 auf einem Boden steht. Vorzugsweise ist der Operationstisch vollständig elektrisch von einer Umgebung isoliert. Vorliegend fließt der von der Aktivelektrode 16 in das Gewebe des Patienten 12 eingespeiste Strom über die Neutralelektrode 18 zurück an den geerdeten Hochfrequenzgenerator (als Masse im elektrischen Sinne).

In dem in Fig. 1 gezeigten Fall fließt allerdings nicht der gesamte durch die Aktivelektrode 16 in den Körper des Patienten eingespeiste Strom über die Neutralelektrode ab, da es, bspw. aufgrund einer unsachgemäßen Handhabung seitens eines Operationspersonal bei der Vorbereitung der Operation oder während dieser, zur Ausbildung einer weiteren Leitungsbrücke 26 an einem Unterschenkel 28 des Patienten kommt. Die Leitungsbrücke 26 ist vereinfacht als schwarzer Fleck auf dem Unterschenkel 28 des Patienten 12 angedeutet. Durch einen unkontrollierten Stromfluss über diese Leitungsbrücke 26, die neben der Neutralelektrode 18 stromseitig eine weitere Abflussmöglichkeit darstellt, kann es zu Verbrennungen an dem betroffenen Bereich des Unterschenkels 28 kommen.

Zur Vorhersage bzw. zur frühzeitigen Erkennung einer Ausbildung solch parasitärer Leitungsbrücken weist das Hochfrequenz-Chirurgie-Systems 100 ein Ausführungsbeispiel der Überwachungseinheit 10 auf

Die Überwachungseinheit 10 weist Messelektroden 30 auf. Vorliegend sind vereinfacht lediglich drei der Vielzahl von Messelektroden 30 gekennzeichnet. Die Messelektroden 30 sind vorliegend in einer unmittelbaren Peripherie des Patienten 12 angeordnet. Die periphere Anordnung der Messelektroden 30 beschränkt sich im vorliegenden Fall darauf, dass die Messelektroden 30 unmittelbar auf einer Auflage-Oberfläche 32 der Patientenliege 22 angeordnet sind. Die Messelektroden 30 sind vorzugsweise jeweils voneinander elektrisch isoliert. Eine zwischen den Messelektroden abfallende Impedanz entsteht durch Stromfluss des Mess-Wechselstroms durch das zwischen jeweils zwei Messelektroden liegende Gewebe des Pateinten 12. Die Messelektroden 30 sind in unmittelbarem Kontakt mit einer Haut des Patienten 12, der auf der Patientenliege 22 liegt.

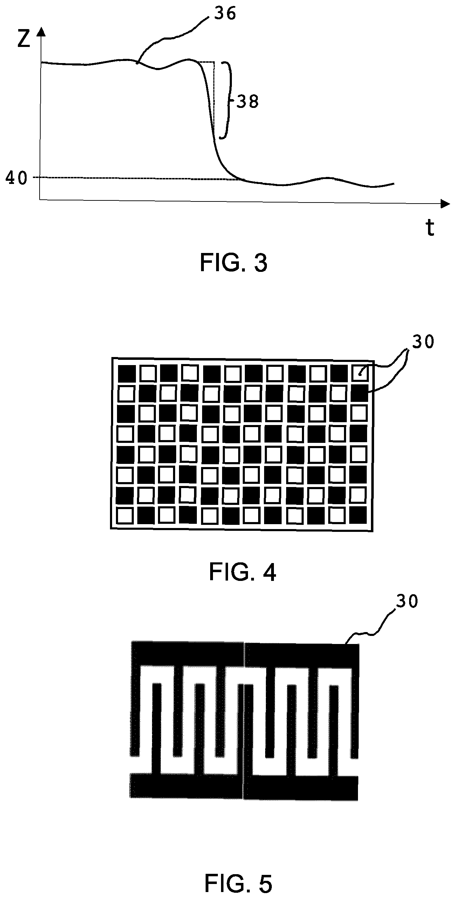

Ferner weist die Überwachungseinheit 10 eine Auswerte- und Steuereinheit 34 auf. Die Auswerte- und Steuereinheit 34 ist dazu eingerichtet, eine vorbestimmte Mess-Wechselspannung oder einen vorbestimmten Mess-Wechselstrom in die Messelektroden 30 einzuprägen. Ferner ist die Auswerte- und Steuereinheit 34 dazu eingerichtet, eine zwischen den Messelektroden 30 abfallende Impedanz Z (bzw. einen Imaginärteil X und/oder Realteil R) zu messen bzw. über Strom- oder Spannungsmessung zu bestimmen. Ferner ist die Auswerte- und Steuereinheit 34 dazu eingerichtet, einen zeitlichen Verlauf der Impedanz 36 (siehe Fig. 3) und/oder eine zeitliche Änderung (∂Z/∂t) derselben zu überwachen und ein akustisches, optisches und/oder taktiles Warnsignal zu erzeugen, wenn eine Relativänderung der Impedanz (∂Z/∂t) in dem zeitlichen Verlauf der Impedanz 36 einen vorbestimmten ersten Grenzwert 38 unterschreitet oder überschreitet und/oder die Impedanz einen vorbestimmten zweiten Grenzwert 40 unterschreitet (vergleiche Fig. 3, in der auf der Abszisse die Zeit t in [s], auf der Ordinate die Impedanz Z in [Ohm] aufgetragen ist).

Vorliegend wird das Warnsignal auf einer Anzeige 42 bzw. einem Display angezeigt, der mit der Auswerte- und Steuereinheit 34 über ein oder mehrere Kabel verbunden ist. Die Anzeige ist an einem Operationsterminal 44 angeordnet, über das der Patient 12 während der Operation mittels eines Beatmungsschlauches 46 beatmet wird.

In Fig. 2 ist ein zweites Ausführungsbeispiel des Hochfrequenz-Chirurgie-Systems 100 mit einem zweiten Ausführungsbeispiel der Überwachungseinheit 10 gezeigt. Das Hochfrequenz-Chirurgiegerät 14 ist in Fig. 2 als bipolare Bauweise eines Elektroskalpells ausgeführt. Bei der bipolaren Bauweise sind Aktivelektrode 16 und Neutralelektrode 18 bspw. in einer Art Pinzette mit zwei voneinander isolierten Schenkeln angeordnet, wobei sich die Aktiv-Neutral-Zuordnung während des Betriebes zwischen den Schenkeln tauschen kann.

Die Überwachungseinheit 10 weist Messsensoren 48 auf. Die Messsensoren 48 sind in der Peripherie des Patienten 12 angeordnet. Vorliegend sind die Messsensoren 48 über die gesamte Oberfläche 32 der Patientenauflage 22 jeweils voneinander beabstandet angeordnet. Ferner sind weitere Messsensoren dem Beatmungsschlauch 46 angeordnet. Die Messsensoren 48 sind dazu eingerichtet, einen Parameter zu erfassen, der während des Trennens und/oder Koagulierens des biologischen Gewebes aufgrund der Hochfrequenz-Energie hervorgerufen wird. Die Auswerte- und Steuereinheit 34 ist dazu eingerichtet, auf Basis des Parameters ein Warnsignal zu erzeugen.

Vorliegend sind die Messsensoren 48 Temperatursensoren, insbesondere thermoelektrische Elemente, wie z. B. Thermo-Elemente. Der gemessene Parameter ist eine Temperaturdifferenz in Kelvin. Kommt es zum Auftreten eines parasitären Stromflusses über eine weitere Leistungsbrücke (siehe hierzu Fig. 1), kann über die Temperatursensoren 48 eine Erwärmung in Form einer Differenztemperatur gemessen werden. Überschreitet die zeitlich gemessene Temperaturdifferenz einen ersten Schwellwert, in Form einer Maximaltemperatur T_{Max}wird das Warnsignal ausgelöst.

In den Fig. 4 und 5 sind zwei beispielhafte Ausführungen von vorteilhaften Anordnungen von Messelektroden 30 dargestellt. Fig. 4 zeigt eine schachbrettartige Anordnung von schwarz und weiß dargestellten Messelektroden 30. Die Impedanz kann jeweils zwischen zwei benachbarten Elektroden gemessen werden, jedoch grundsätzlich auch zwischen zwei belieben positiv-negativ-Elektrodenpaaren in der Schachbrettanordnung, bei der die Messelektroden 30 ein Muster aus räumlich verteilten, jeweils benachbarten und vorzugsweise voneinander elektrisch isolierten Flächenelektroden bilden. In Fig. 5 ist eine sogenannte Interdigital-Struktur dargestellt.

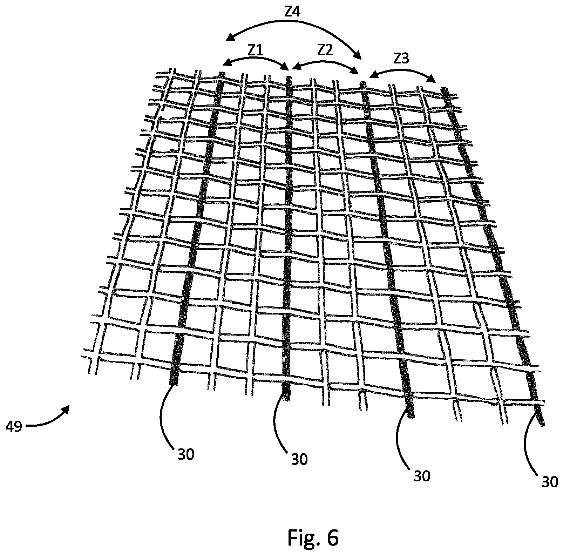

Fig. 6 zeigt ein Ausführungsbeispiel von Messelektroden 30, die bspw. in Form von metallischen Fäden, in einem Gewebe eingearbeitet, z. B. eingewebt sind. Das dargestellte Gewebe 49 kann bspw. aus nichtleitendem Stoff oder Polyester hergestellt sein und eine Vielzahl von miteinander verwobenen und/oder verflochtenen Fäden aufweisen. Einige dieser Fäden dienen in metallischer bzw. elektrisch leitfähiger Ausführung (z. B. auf Kohlenstoffbasis) als Messelektroden 30. Aus einem derartigen Stoff kann beispielsweise ein Sitz- oder Liegebezug für die Patientenauflage 22 hergestellt sein. Alternativ kann aus einem derartigen Gewebe auch eine (Auflage-) Matte hergestellt sein. Je nach Art der Einwebung der metallischen Fäden bzw. Messelektroden kann auch eine Schachbrettmuster-Anordnung der Elektroden hergestellt werden.

In Fig. 6 sind schematische Impedanz-Messungen als Pfeile zwischen den jeweiligen Messelektroden 30 eingezeichnet. Die Impedanzen Z₁ bis Z₄ werden vorliegend indirekt über eine Strom- oder Spannungsmessung durch die Auswerte- und Steuereinheit 20 gemessen. Die Impedanz Z₄ wird nicht direkt zwischen zwei benachbarten Messelektroden 30 gemessen, sondern zwischen den Messelektroden 30 wurde eine Messelektrode übersprungen.

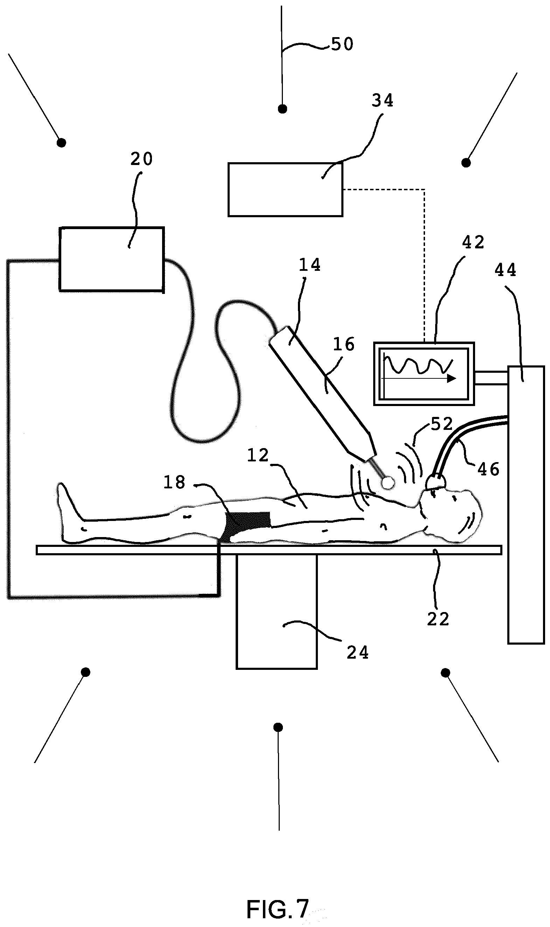

Fig. 7 zeigt ein drittes Ausführungsbeispiel des Hochfrequenz-Chirurgie-Systems 100 mit einem dritten Ausführungsbeispiel der Überwachungseinheit 10. Die Überwachungseinheit 10 weist als Messsensoren 48 jeweils magnetische und/oder elektrische Antennen 50 auf. Die Antennen 50 sind dazu eingerichtet, als den Parameter jeweils elektromagnetische Signale 52 aus der Peripherie des Patienten 12 zu erfassen, die durch die Hochfrequenz-Energie während des Betriebes des Hochfrequenz-Chirurgiegerätes 14 erzeugt werden.

Die Auswerte- und Steuereinheit 34 ist dazu eingerichtet, über Lösen einer mathematischen Inversproblematik aus den Messsignalen eine Stromdichteverteilung im Raum zu berechnen, wobei sich die erfassten Messsignale bei magnetischen Antennen 50 proportional zu 1/Abstand • ∂l/∂t, bei elektrischen Antennen 50 proportional zu 1/Abstand • ∂V/∂t verhalten. Die Antennen 50 sind vorzugsweise in einer unmittelbaren Operationsumgebung, bspw. an der Patientenauflage 22 und/oder an einer Decke über dem Operationsgeschehen und/oder in Ecken des Operationssaales angeordnet. Vorliegend sind sechs Antennen 50 schematisch dargestellt, von denen aus Gründen der Übersichtlichkeit lediglich eine gekennzeichnet wurde.

Zu Illustrationszwecken beschrieben, aber nicht als solche beansprucht werden im folgenden einige Verfahren zum Überwachen eines Patienten. Besonders bevorzugt ist ferner ein Verfahren zum Überwachen eines Patienten 12 während eines Betriebes eines Hochfrequenz-Chirurgiegerätes 14, wobei das Hochfrequenz-Chirurgiegerät 14 dazu ausgebildet ist, biologisches Gewebe mittels elektrischer Hochfrequenz-Energie zu trennen und/oder zu koagulieren. Das Verfahren weist die Schritte auf: Einprägen einer vorbestimmten Mess-Wechselspannung oder eines vorbestimmten Mess-Wechselstromes in Messelektroden 30, die in einer Peripherie des Patienten 12 angeordnet sind, und Messen einer zwischen den Messelektroden 30 abfallende Impedanz, sowie Überwachen eines zeitlichen Verlaufs der Impedanz 36 und/oder einer zeitlichen Änderung derselben.

Ebenfalls bevorzugt ist als Alternativlösung ferner ein Verfahren zum Überwachen eines Patienten 12 während eines Betriebes eines Hochfrequenz-Chirurgiegerätes 14, wobei das Hochfrequenz-Chirurgiegerät 14 dazu ausgebildet ist, biologisches Gewebe mittels elektrischer Hochfrequenz-Energie zu trennen und/oder zu koagulieren. Das Verfahren weist die Schritte auf: Erfassen von elektromagnetischen Messsignalen aus einer Peripherie des Patienten mittels magnetischer und/oder elektrischer Antennen, wobei die elektromagnetischen Messsignale durch die Hochfrequenz-Energie während des Betriebes des Hochfrequenz-Chirurgiegerätes erzeugt werden; und Berechnen einer Stromdichteverteilung im Raum (bzw. von dem Patienten und dessen Peripherie) aus den elektromagnetischen Mess-signalen durch Lösen einer mathematischen Inversproblematik.

Die bevorzugten Verfahren zum Überwachen eines Patienten 12 während eines Betriebes eines Hochfrequenz-Chirurgiegerätes 14 können gemäß der vorstehend offenbarten Ausgestaltungen und Ausführungsformen in einer jeweiligen verfahrensspezifischen Abwandlung ausgestaltet sein, ohne an dieser Stelle in redundanter Form erwähnt zu werden.

## Patentansprüche

1. Überwachungseinheit, die dazu eingerichtet ist, einen Patienten (12) während eines Betriebes eines Hochfrequenz-Chirurgiegerätes (14) zu überwachen, wobei das Hochfrequenz-Chirurgiegerät (14) dazu ausgebildet ist, biologisches Gewebe mittels Hochfrequenz-Energie zu trennen und/oder zu koagulieren, wobei die Überwachungseinheit (10) aufweist:
- Messsensoren (48), und
- eine Auswerte- und Steuereinheit (34),
wobei die Messsensoren (48) dazu eingerichtet sind, einen Parameter zu erfassen, der während des Trennens und/oder Koagulierens des biologischen Gewebes aufgrund der Hochfrequenz-Energie hervorgerufen wird,
**dadurch gekennzeichnet, dass** die Messsensoren (48) jeweils magnetische und/oder elektrische Antennen (50) sind, die dazu eingerichtet sind, als den Parameter jeweils elektromagnetische Messsignale aus der Peripherie des Patienten (12) zu erfassen, die durch die Hochfrequenz-Energie während des Betriebes des Hochfrequenz-Chirurgiegerätes (14) erzeugt werden, und
wobei die Auswerte- und Steuereinheit (34) dazu eingerichtet ist, mittels Lösens einer mathematischen Inversproblematik aus den elektromagnetischen Messsignalen eine Stromdichteverteilung im Raum zu berechnen.

2. Überwachungseinheit nach Anspruch 1, die ferner dazu ausgebildet ist, bei Überschreiten einer vorbestimmten Schwelle ein Warnsignal zu erzeugen.

3. Überwachungseinheit nach Anspruch 2, wobei das Warnsignal in akustischer, optischer und/oder taktiler Form ausgebbar ist.

4. Überwachungseinheit nach einem der vorstehenden Ansprüche, wobei die errechnete dreidimensionale Stromdichteverteilung bildhaft darstellbar ist.

5. Überwachungseinheit nach Anspruch 4, wobei die bildhaft darstellbare dreidimensionale Stromdichteverteilung mit Bildern einer 3D-Stereo-Kamera derart überlagerbar ist, dass die Stromdichte-Informationen an einer teiltransmissiven 3D-Abbildung des Patienten visualisierbar sind.

6. Überwachungseinheit Anspruch 2, wobei die Auswerte- und Steuereinheit dazu eingerichtet, das Warnsignal zu erzeugen und/oder das Hochfrequenz-Chirurgiegerät abzuschalten, wenn die Stromdichte einer oder mehreren vorbestimmten Positionen im Raum einen vorbestimmten zweiten Schwellwert überschreitet.

7. Überwachungseinheit Anspruch 6, wobei die eine oder mehreren Positionen ein Teil oder mehrere Teile eines Rückens des Patienten, mit welchen der Patient auf der Operationsliege aufliegt, und/oder ein oder mehrere Teile eines Fußes oder einer sonstigen Gliedmaße des Patienten repräsentieren.

8. Hochfrequenz-Chirurgie-System, das aufweist:
- einen Hochfrequenz-Generator (20), der dazu eingerichtet ist, eine Hochfrequenz-Energie zu erzeugen,
- ein Hochfrequenz-Chirurgiegerät (14) mit einer Aktivelektrode (16) und einer Neutralelektrode (18), wobei zumindest die Aktivelektrode (16) mit dem Hochfrequenz-Generator (20) verbunden ist,
wobei die Aktivelektrode (16) dazu ausgebildet ist, biologisches Gewebe mittels der elektrischen Hochfrequenz-Energie zu trennen und/oder zu koagulieren, und
wobei die Neutralelektrode (18) dazu eingerichtet ist, die elektrische Hochfrequenz-Energie aus dem biologischen Gewebe abzuleiten, und
- eine Überwachungseinheit (10) nach einem der Ansprüche 1-7.

## Claims

1. A monitoring unit, which is configured to monitor a patient (12) during operation of a high-frequency surgery device (14), wherein the high-frequency surgery device (14) is configured to separate and/or coagulate biological tissue by means of high-frequency energy, wherein the monitoring unit (10) has:
- measuring sensors (48), and
- an evaluation and control unit (34),
wherein the measuring sensors (48) are configured to detect a parameter which is caused during the separation and/or coagulation of the biological tissue because of the high-frequency energy
**characterized in that** the measuring sensors (48) are each magnetic and/or electric antennas (50) which are configured to detect electromagnetic measuring signals from the periphery of the patient (12) as the parameter, the measuring signals being generated by the high-frequency energy during operation of the high-frequency surgery device (14), and
wherein the evaluation and control unit (34) is configured to calculate a spatial distribution of electric current density from the electromagnetic measuring signals by solving a mathematical inverse problem.

2. The monitoring unit according to claim 1, which is further configured to generate a warning signal upon exceeding a predetermined limit value.

3. The monitoring unit according to claim 2, wherein the warning signal is emitted in acoustic, optical and/or tactile form.

4. The monitoring unit according to any one of the preceding claims, wherein the calculated three-dimensional spatial distribution of electric current density can be visually depicted.

5. The monitoring unit according to claim 4, wherein the visually depicted three-dimensional spatial distribution of electric current density can be superposed with images of a 3D stereo camera such that the information on the electric current density is visualizable on a partially transmissive 3D depiction of the patient.

6. The monitoring device according to claim 2, wherein the evaluation and control unit is configured to generate the warning signal and/or to shut down the high-frequency surgery device if the electrical current density of one or more predetermined positions in the space exceeds a second predetermined limit value.

7. The monitoring unit according to claim 6, wherein the one or several positions represent a part or several parts of the patient's back, on which the patient lies on the operating table, and/or a part or several parts of the patient's foot or other extremities.

8. A high-frequency surgery system which has:
- a high-frequency generator (20) which is configured to generate a high-frequency energy,
- a high-frequency surgery device (14) having an active electrode (16) and a dispersive electrode (18), wherein at least the active electrode (16) is connected to the high-frequency generator (20),
wherein the active electrode (16) is configured to separate and/or coagulate biological tissue by means of the high-frequency electrical energy, and
wherein the dispersive electrode (18) is configured to discharge the high-frequency electrical energy from the biological tissue, and
- a monitoring unit (10) according to any one of claims 1-7.

## Revendications

1. Unité de surveillance, qui est configurée pour surveiller un patient (12) pendant le fonctionnement d'un dispositif (14) de chirurgie à haute fréquence, dans laquelle le dispositif (14) de chirurgie à haute fréquence est configuré pour séparer et/ou coaguler le tissu biologique au moyen d'une énergie à haute fréquence, dans laquelle l'unité de surveillance (10) a :
- des capteurs de mesure (48), et
- une unité (34) d'évaluation et de contrôle,
dans laquelle les capteurs de mesure (48) sont configurés pour détecter un paramètre qui est causé pendant la séparation et/ou la coagulation du tissu biologique à cause de l'énergie à haute fréquence **caractérisée en ce que** les capteurs de mesure (48) sont chacun des antennes (50) magnétiques et/ou électriques qui sont configurées pour détecter des signaux de mesure électromagnétiques provenant de la périphérie du patient (12) en tant que paramètre, les signaux de mesure étant générés par l'énergie à haute fréquence pendant le fonctionnement du dispositif (14) de chirurgie à haute fréquence, et
dans laquelle l'unité (34) d'évaluation et de contrôle est configurée pour calculer une distribution spatiale de la densité de courant électrique à partir des signaux de mesure électromagnétiques en résolvant un problème mathématique inverse.

2. Unité de surveillance selon la revendication 1, qui est en outre configurée pour générer un signal d'avertissement en cas de dépassement d'une valeur de seuil prédéterminée.

3. Unité de surveillance selon la revendication 2, dans laquelle le signal d'avertissement est émis sous forme acoustique, optique et/ou tactile.

4. Unité de surveillance selon l'une quelconque des revendications précédentes, dans laquelle la distribution spatiale tridimensionnelle calculée de la densité de courant électrique peut être représentée visuellement.

5. Unité de surveillance selon la revendication 4, dans laquelle la distribution spatiale tridimensionnelle de la densité du courant électrique, représentée visuellement, peut être superposée aux images d'une caméra stéréo 3D, de sorte que les informations sur la densité du courant électrique sont visualisables sur une représentation 3D partiellement transmissive du patient.

6. Unité de surveillance selon la revendication 2, dans laquelle l'unité d'évaluation et de contrôle est configurée pour générer le signal d'avertissement et/ou pour arrêter le dispositif de chirurgie à haute fréquence si la densité de courant électrique d'une ou plusieurs positions prédéterminées dans l'espace dépasse une deuxième valeur de seuil prédéterminée.

7. Unité de surveillance selon la revendication 6, dans laquelle la ou les positions représentent une ou plusieurs parties du dos du patient, sur lequel le patient est allongé sur la table d'opération, et/ou une ou plusieurs parties du pied ou d'autres extrémités du patient.

8. Système de chirurgie à haute fréquence qui a :
- un générateur (20) à haute fréquence configuré pour générer une énergie à haute fréquence,
- un dispositif (14) de chirurgie à haute fréquence ayant une électrode active (16) et une électrode dispersive (18), dans lequel au moins l'électrode active (16) est connectée au générateur (20) à haute fréquence,
dans laquelle l'électrode active (16) est configurée pour séparer et/ou coaguler le tissu biologique au moyen de l'énergie électrique à haute fréquence, et
dans laquelle l'électrode dispersive (18) est configurée pour décharger l'énergie électrique à haute fréquence du tissu biologique, et
- une unité de surveillance (10) selon l'une quelconque des revendications 1 à 7.
